(19) Europäisches Patentamt
European Patent Office
Office européen des brevets

(11) Publication number: **0 123 902 B1**

# (12) EUROPEAN PATENT SPECIFICATION

(45) Date of publication of patent specification: **18.09.91** (51) Int. Cl.$^5$: **C12Q 1/28**

(21) Application number: **84103368.1**

(22) Date of filing: **27.03.84**

## (54) Procedure for the determination of peroxidase activity by end dilution titration and means for its realization.

(30) Priority: **31.03.83 DE 3311887**

(43) Date of publication of application:
**07.11.84 Bulletin 84/45**

(45) Publication of the grant of the patent:
**18.09.91 Bulletin 91/38**

(84) Designated Contracting States:
**AT BE CH DE FR GB IT LI LU NL SE**

(56) References cited:
**EP-A- 0 029 917**
**DE-A- 2 811 228**
**GB-A- 981 955**
**EP-A- 0 123 901**
**FR-A- 2 295 425**
**US-A- 2 905 594**

(73) Proprietor: **Byk-Sangtec Diagnostica GmbH & Co. KG**
**Postfach 2060 Von-Hevesy-Strasse 3**
**W-6057 Dietzenbach(DE)**

(72) Inventor: **Lentfer, Dierck, Dr.**
**Karpfenweg 3**
**W-6054 Rodgau 6(DE)**

(74) Representative: **RUPP, Herbert Byk Gulden Lomberg Chemische Fabrik GmbH et al**
**Byk-Gulden-Strasse 2 Postfach 6500**
**W-7750 Konstanz(DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The invention concerns a procedure for the determination of peroxidase activity and a reagent composition for its determination.

In EP-A 0029917 a qualitative method for the detection of small amounts of peroxidase is described. The method is based on the known color appearance connected with the reaction of hydrogen peroxide with a benzidine type indicator in the presence of peroxidase. According to EP-A 0029917 the sensitivity of the test can be enhanced by the addition of an amine oxide.

Peroxidase, e.g. horse radish peroxidase (HRPO) finds broad application as a marker enzyme in enzyme-immunological determination methods (Enzyme immuno assay (EIA)). A common feature of all EIA systems is the exploitation of an antigen-antibody-interaction for the determination of the concentration of one of the reactants (antigen or antibody). A multitude of EIA systems have been described. These may be classified in systems with enzyme labelled antigen and in systems with enzyme labelled antibody.

One example of an EIA system with labelled antigen is the competitive solid phase EIA (Enzyme-linked Immuno Sorbent Assay (ELISA)). In this a known amount of enzyme-labelled antigen and the antigen to be determined (analyte) compete for the limited number of binding sites of a specific antibody which is immobilized on a solid phase. The higher the concentration of analyte, the lower the amount of enzyme-labelled antigen bound to the immobilized antibody. Making use of a calibration curve, the determination of the enzyme activity bound to the the solid phase by interaction with the antibody allows the determination of the analyte concentration.

An exponent of the antibody-labelled EIA is the "Sandwich"-assay (or two-site enzyme immunometric test). The test includes two immunological reactions: In the first reaction a macromolecular antigen is bound more or less completely to the antibody immobilized on a solid phase. After removal of the reaction solution, the bound antigen is reacted with an excess of a conjugate consisting of a specific antibody and enzyme. The extent of the binding of enzyme-labelled antibody is proportional to the amount of antigen, thus the determination of the remaining enzyme activity after removal of excess conjugate is a measure of the amount of antigen.

Thus, the determination of enzyme activity is an essential step in all EIA's. In general, enzyme activity is determined by well known procedures, typically by following the changes of the optical properties (absorption, fluorescence, luminescence) of an appropriate substrate effected by enzymatic action. Appropriate measuring instruments are used, and a linear proportionality between measuring signal and amount of enzyme is advantageous. For the determination of peroxidases, benzidine and its derivatives as o-dianisidine, o-tolidine, dicarboxidine and 3,3',5,5'-tetramethylbenzidine in combination with hydrogen peroxide are useful. The reference Z.med.Labor.-Diagn. 24 155-160 (1983) relates to the use of o-dianisidine or o-phenylenediamine as hydrogen donors in an automatized kinetic method for determination of horse radish peroxidase.

For a variety of applications of EIA, e.g. in serological determinations of antibody-titers, the high precision achievable by instrumental measurement is only of little value, since only rises in concentration by a factor of 2 to 4 relative to a reference are considered meaningful, whereas the whole concentration range to be covered includes several orders of magnitude. For such determinations the method of end dilution titration is applicable. It consists in the determination of the highest dilution of the original solution which gives a positive result for the analyte of interest in a defined detection procedure. The reciprocal of this highest dilution (e.g. 1:1,000) is usually denoted as "titer" (e.g. 1,000). The numerical value is of course influenced by the sensitivity of the detection method in use which may be different from case to case due to fluctuations in the performance. This can be overcome by standardization of the titers against a reference of known or defined concentration which is conducted in every determination.

In practice the sample solution is diluted serially with an appropriate diluent, normally in geometrical series (e.g. titer steps 1:10; 100; 1,000 ... or 1:2; 4; 8; ... or 1:1.5; 2; 3; 4; 6...). A variety of simple technical means are available for this purpose, e.g. the microtiter system. An equal volume of each titer step is subjected to the test reaction which in form of a yes-no answer leads to the identification of the highest titer. If a visual observable detection reaction is applied, no measuring instrument is needed.

The applicability of a detection reaction is highly dependent upon the unambiguity with which a (weakly) positive result may be discerned from a negative result (yes-no-answer). But the detection of enzyme activities with chromogenic substrates usually leads to continuous increase (decrease) in color development with rising (falling) enzyme concentration, thus making almost impossible the clear-cut identification of dilution end points.

The objective of the present invention is to provide a detection reaction for peroxidase which unambiguously indicates the surpassing (subpassing) of a certain amount of enzyme by an abrupt change

in a visual observable property (color jump) and thus allows the unambiguous detection of end point titrations, i.e the clear-cut demonstration of the sub- or surpassing of a threshold concentration, and to provide a composition for the performance of this reaction.

The object is fulfilled by providing an improved method for the determination of peroxidase activity by end dilution titration under peroxidase end point dilution titration determining conditions with a benzidine derivative in the presence of a color retarding amount of non-chromogenic or low-chromogenic proton donor to retard color development for a sufficient period of time to permit a sharp dilution endpoint and thereafter detecting peroxidase activity.

Peroxidases of different origins may be determined, e.g. Japanese radish peroxidase, especially horse radish peroxidase (HRPO) (EC1,11.1.7.).

Benzidine derivatives are those of the general formula (I)

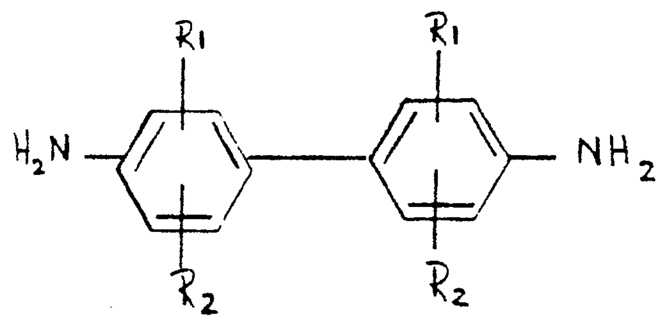

where

R1 stands for a hydrogen atom, a halogen atom, an amino group, a ($C_1$-$C_4$) alkyl group, a ($C_1$-$C_4$) alkoxy group, a carboxy group, a sulfo group or a carboxypropyloxy group and where

R2 stands for a hydrogen atom, a halogen atom, a ($C_1$-$C_4$) alkyl group, or the salts thereof.

($C_1$-$C_4$) alkyl groups are branched or unbranched alkyl groups, likewise the ($C_1$-$C_4$) alkoxy groups maybe branched or unbranched.

The methyl and methoxy group are preferred. Halogen atoms include bromine, chlorine and fluorine; chlorine is preferred.

Compounds of formula (I), where R1 stands for a hydrogen atom, a methyl group, or a methoxy group and where R2 stands for a hydrogen atom or a methyl group, are preferred.

Especially preferred is the compound of formula (I), where R1 and R2 stand for a methyl group in the ortho position to the amino groups.

Non-chromogenic or low-chronogenic proton donors are phenylene derivatives of the general formula (II)

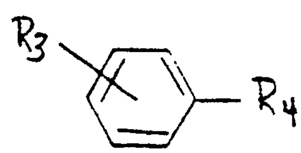

where

R3 and R4 are equal or different and stand for a hydroxy group, an amino group, a ($C_1$-$C_4$) alkyl amino group or a benzylamino group.

Preferred examples of the phenylene derivatives are hydroquinone, 1,2-dihydroxybenzene, resorcinol, o-, m-and p-phenylene diamine, 4-aminophenol, 4-methylaminophenol, 4-benzyl amino phenol. Selected phenylene derivatives (II) are o-phenylendiamine and 4-aminophenol, especially hydroquinone.

As mentioned, procedures for determining peroxidase activity by end dilution titration are well known and are carried out under peroxidase end dilution titration determining conditions. Likewise, the improved procedure of this invention is carried out under peroxidase activity end dilution titration determining conditions utilizing a sufficient amount of the benzidine derivative, usually an excess amount of hydrogen peroxide or its adducts. The hydrogen peroxide can be added to the reaction or generated in situ. Such conditions include suitable reaction temperatures, e.g. 4 °C to 37 °C and the use of a buffer, e.g. TRIS to maintain the pH from about 4.5 to 7 during the determination. The appropriate amount of benzidine

derivative can be determined by routine experimentation as is known for peroxidase determinations, for example, it can range from 0.01 to 0.2 mg per ml of the test sample containing the peroxidase enzyme.

In accordance with the invention, the phenylene derivative II is present during the peroxidase benzidine derivative reaction. It is used in color retarding amounts such that color development is retarded for a sufficient period of time to permit sharp end point determinations of the absence or presence of color, for example, it can be used in an amount that ranges from about 0.0005 to 0.01 mg per ml of test sample containing the peroxidase enzyme. Expressed another way, the phenylene derivative is present in an amount from about 0.1 to 10 weight percent of benzidine derivative I.

The admixture of phenylene derivative II does not influence the color absorbance peak. The admixture of II to the reaction system I/$H_2O_2$/peroxidase results in a retardation of the onset of color development, the duration of which is directly proportional to the amount of II added and is inversely proportional to the amount of enzyme. During the retardation phase, color development is completely suppressed. After the onset of color development, its rate is the same as that observed without admixture of II under the same conditions. The visible range of the absorption spectrum remains qualitatively unchanged.

The combination of the enzyme-dependent retardation of color development with a fixed reaction time for observation of color results in the desired ability to answer the passing of a threshold concentration by an unambiguous yes/no signal, in this case: colored/not colored.

The invention may be further illustrated using as an example the combination of 3,3',5,5'-tetramethylbenzidine (TMB) with hydroquinone for the determination of HRPO.

With TMB the maximum absorption peak for the blue color is at 645 nm. This is not altered by the admixture of hydroquinone.

However, admixture of hydroquinone to the reaction system TMB:$H_2O_2$:HRPO results in the retardation of the onset of color development in the entire visible range of the spectrum (700-300 nm), the duration of which is directly proportional to the amount of hydroquinone added and is inversely proportional to the amount of HRPO (Diagrams 1 and 2).

During the retardation phase the color development is completely suppressed, after the onset of color development its rate is the same as that observed without addition of hydroquinone under the same conditions.

The retardation in the onset of blue color development which is inversely proportional to the amount of HRPO in combination with a fixed reaction time for observance of color results in the desired ability to answer the passing of a threshold concentration by an unambiguous yes/no signal; in this case: blue/not blue. This is illustrated by the experiment shown in table 1 (the photometrically obtained absorbance values serve only for the exemplification of the effect which, according to the invention, allows the visual, non-instrumental evaluation of end dilution titrations).

Starting with $1.1 \cdot 10^{-9}$ g HRPO per ml of substrate mixture two parallel dilution series with the dilution factors 1; 1.5; 2; 3; 4; 6; ... 128; 192 are prepared. By incubation with a substrate mixture containing only TMB as a proton donor in higher dilutions of the enzyme ($A_{645,\ 1cm} \leqq 1$) a gradually decreasing blue color is obtained which is directly proportional to the amount of HRPO. The identification of a dilution end point is not possible. If under the same conditions a substrate mixture is used which contains a small amount of hydroquinone, a dilution end point can be identified without doubt (highest dilutions with visually observable blue color: 1:8 after 30 minutes; 1:12 after 60 minutes).

Table 1

$OD_{645}$ (Optical Density at 645 nm)

| Dilution Factor (Start: $1.1 \cdot 10^{-9}$g HRPO | TMB 30min | TMB 60min | TMB + Hydroquinone 30min | TMB + Hydroquinone 60 min |
|---|---|---|---|---|
| 1 | >2 | >2 | >2 | >2 |
| 1.5 | >2 | >2 | >2 | >2 |
| 2 | >2 | >2 | >2 | >2 |
| 3 | >2 | >2 | >2 | >2 |
| 4 | >2 | >2 | >2 | >2 |
| 6 | 1.69 | >2 | 0.768 | 1.35 |
| 8 | 1.36 | 1.83 | 0.394 | 0.90 |
| 12 | 0.90 | 1.26 | 0.015 | 0.307 |
| 16 | 0.718 | 0.997 | 0.001 | 0.022 |
| 24 | 0.499 | 0.699 | 0 | 0 |
| 32 | 0.370 | 0.525 | 0 | 0 |
| 48 | 0.244 | 0.353 | 0 | 0 |
| 64 | 0.185 | 0.268 | 0 | 0 |
| 96 | 0.149 | 0.179 | 0 | 0 |
| 128 | 0.099 | 0.143 | 0 | 0 |
| 192 | 0.063 | 0.093 | 0 | 0 |

The threshold concentration of the enzyme may be adapted to the requirement by proper choice of the two parameters: Concentration of phenylene derivative II and reaction time to give suitable color development.

A shift of the end point to an amount of enzyme lowered by the factor 1.5 would increase the reaction time necessary by at least the same factor.

If reaction time and determination of end point are properly chosen (e.g. 30 min.), a deviation from this (e.g. reaction time between 24 and 36 min.) will lead to results different in one titer step at worst (factor: 1.5).

Reaction time and end point determination will adequately be in a range where color development is sufficient, i.e. between 5 and 60 minutes.

The unambiguity of the end point determination is exchanged for a small loss in the sensitivity of detection. But this is of no importance in many fields of application of EIA's with visual evaluation or may be

compensated for by an adequate assay design.

The combination of favourable properties which are offered by the principle of end dilution titration concerning low cost and high flexibility of instrumentation needed and wide measuring range with the virtues of enzyme-immuno-logical methods (specifity, sensitivity, universality of detection reaction) is very desirable.

Results corresponding to those described here in detail for the combination TMB-hydroquinone are obtained with other combinations of benzidine derivatives I and phenylene derivatives II. This is true for the combination TMB/p-phenylendiamine TMB/m-phenylene diamine; TMB/1.2-dihydroxybenzene, o-tolidine/p-phenylene diamine; o-tolidine/m-phenylene diamine, o-tolidine/1.2-dihydroxy-benzene and o-tolidine/4-aminophenol as especially for the combinations

- TMB/hydroquinone; TMB/4-aminophenol
- TMB/o-phenylene diamine;
  o-tolidine/hydroquinone;
- o-tolidine/o-phenylendiamine;
  o-dianisidine/hydroquinone and
- benzidine/hydroquinone.

This invention also relates to a reagent composition for the determination of peroxidase by end point dilution, containing a benzidine derivative of the general formula I in a sufficient amount to permit the determination of peroxidase activity by color formation and a phenylene derivative of the general formula II in a color retarding amount to prevent color formation for a sufficient period of time to permit a sharp end point determination.

The reagent may contain the usual additives in the usual amounts including hydrogen peroxide or its adducts, e.g. urea or sodium borate-$H_2O_2$ adducts, in an amount sufficient to aid in color formation, buffers e.g. TRIS in amounts to maintain the pH range from 4.5 to 7 during the determination and chelating agents in chelating amounts for the chelating of heavy metals.

Such reagent compositions for determination of peroxidase according to this invention may be distributed commercially as such or in the form of a kit. If they are distributed in the form of a kit, this kit contains the benzidine derivative, the phenylene derivative, usual additives, a peroxidase-labelled antigen or antibody conjugate and a reaction vessel or other solid support which is coated with antigen or antibody.

The working instruction given in the following serves for the illustration of the invention without restricting it.

EXAMPLE 1

Working Instructions (TMB/hydroquinone as an Example)

Chromogen Stock Solutions

1. 10 g 3,3'5,5'-tetramethylbenzidine (analytical grade, free base) are dissolved in dimethylsulfoxide to give 100 ml.
2. 0.1 g hydroquinone is dissolved in dimethylsulfoxide to give 100 ml.

Tris-Citrate-Buffer pH 5
12.1 9 tris(hydroxymethyl) aminomethane and
10.5 g citric acid-1-hydrate are dissolved in distilled water to give 1 liter.

Substrate Mixture (to be prepared daily fresh)
50 ml tris-citrate buffer, 0.05 ml each of the two chromogen stock solutions and 0.1 ml 3% hydrogen peroxide are mixed.

The reactions are started by the quick addition of 1 ml of the respective substrate mixture to 0.01 ml of the respective dilution of horse radish peroxidase. The evaluation of the color change is done after 5 to 60 minutes, preferably after 15 to 45 minutes.

EXAMPLE II

Kit for the Estimation of Antibodies Against Streptolysine-O in Human Sera
The kit includes:

a. Reagents for the determination of peroxidase activity by end dilution titration with visual evaluation, according to the invention:

- a solution of 10 mg/ml 3,3'5,5'-tetramethylbenzidine in dimethylsulfoxide
- a solution of 0.1 mg/ml hydroquinone in dimethylsulfoxide
- a solution of hydrogen peroxide (3%)
- a 0.1 mole per liter solution of tris-citrate-buffer, ph 5

b. A solution of a conjugate of horse radish peroxidase and rabbit IgG, directed against Human IgG.

c. A microtiter plate coated with Streptolysine-O as an antigen-coated reaction vessel.

d. A solution of 0.1 mole per liter tris-HCl buffer, pH 7,8 containing 0.1% Tween® 20; as a diluent for the serum samples to be tested and for antibody-peroxidase-conjugate b.

## Claims

1. In a procedure for the determination of peroxidase activity by end dilution titration under peroxidase end point dilution titration determining conditions wherein a peroxidase is reacted with a benzidine derivative, the improvement comprising conducting said procedure utilizing as the benzidine derivative a benzidine derivative of the general formula (I)

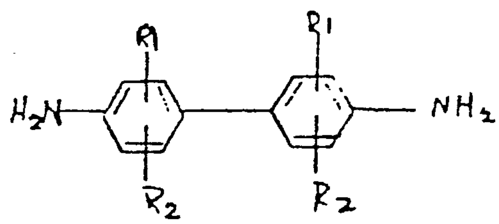

where

R1 stands for a hydrogen atom, a halogen atom, an amino group, a $(C_1-C_4)$ alkyl group, a $(C_1-C_4)$ alkoxy group, a carboxy group, a sulfo group or a carboxypropyloxy group and

where

R2 stands for a hydrogen atom, a halogen atom, a $(C_1-C_4)$ alkyl group, or a salt of this benzidine derivative, in the presence of a non-chromogenic or low-chromogenic phenylene derivative of the general formula (II)

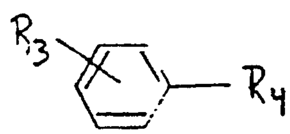

where

R3 and R4 are equal or different and stand for a hydroxy group, an amino group, a $(C_1-C_4)$ alkyl amino group or a benzyl amino group, to retard color development to permit a sharp dilution end point, and thereafter determining peroxidase activity.

2. Procedure according to Claim 1, where R1 of the benzidine derivative I, stands for hydrogen, methyl, or methoxy and where R2 stands for hydrogen or methyl.

3. Procedure according to Claim 1, where R1 and R2 of the benzidine derivative I, stand for methyl in ortho position to the respective amino groups.

4. Procedure according to Claim 1, where the phenylene derivative II is hydroquinone, o-phenylene diamine, or 4-aminophenol.

5. Procedure according to Claim 1, where the phenylene derivative II is hydroquinone.

6. Procedure according to Claim 1, where the color is retarded for a period of 5 to 60 minutes.

7. Procedure according to Claim 6, where the color is retarded for a period of 15 to 45 minutes.

8. A reagent for determining peroxidase activity by end dilution titration comprising a benzidine derivative of the general formula (I)

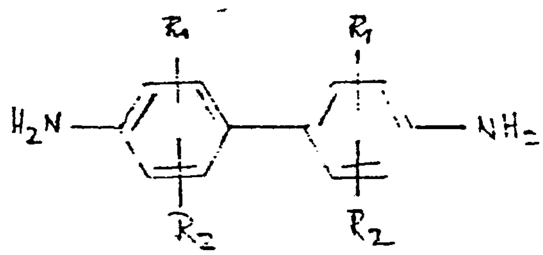

where
R1 stands for a hydrogen atom, a halogen atom, an amino group, a ($C_1$-$C_4$) alkyl group, a ($C_1$-$C_4$) alkoxy group, a carboxy group, a sulfo group or a carboxypropyloxy group and
where
R2 stands for a hydrogen atom, a halogen atom, a ($C_1$-$C_4$) alkyl group, or a salt of this benzidine derivative and a phenylene derivative of the general formula (II)

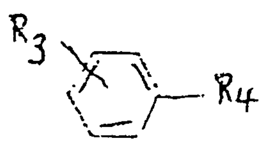

where
R3 and R4 are equal or different and stand for a hydroxy group an amino group, a ($C_1$-$C_4$) alkyl amino group or a benzyl amino group.

9. A kit for determining peroxidase activity by end dilution titration comprising
(a) a benzidine derivative of the general formula (I)

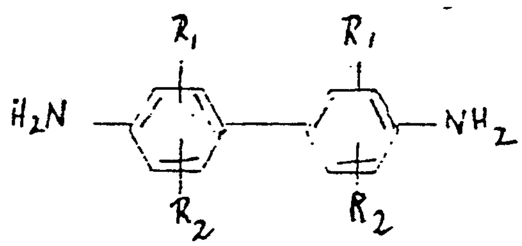

where
R1 stands for a hydrogen atom, a halogen atom, an amino group, a ($C_1$-$C_4$) alkyl group, a ($C_1$-$C_4$) alkoxy group, a carboxy group, a sulfo group or a carboxypropyloxy group,
where
R2 stands for a hydrogen atom, a halogen atom, a ($C_1$-$C_4$) alkyl group, or a salt of this benzidine derivative,
(b) a phenylene derivative of the general formula (II)

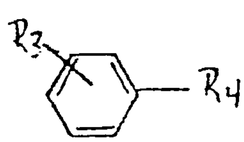

where

R3 and R4 are equal or different and stand for a hydroxy group an amino group, a ($C_1$-$C_4$) alkyl amino group or a benzyl amino group,
(c) hydrogen peroxide or an adduct thereof,
(d) a conjugate of horse radish peroxidase and an antigen or antibody, and
(e) a solid support coated with an antigen or antibody which will react with the analyte to be determined.

## Revendications

1. Dans une procédure de détermination de l'activité peroxydase par titrage à la dilution finale dans des conditions déterminant le titrage de la peroxydase au point final de dilution, dans laquelle une peroxydase est traitée avec un dérivé de benzidine, l'amélioration caractérisée en ce que cette procédure utilise en tant que dérivé de benzidine, un dérivé de benzidine de formule générale (I) :

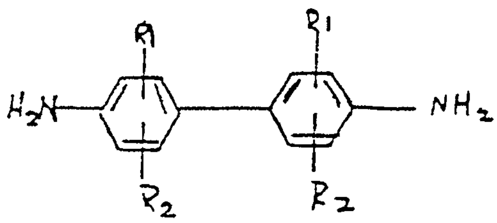

dans laquelle $R_1$ est un atome d'hydrogène, d'halogène, un groupe amino, alkyle en $C_{1-4}$, alcoxy en $C_{1-4}$ , carboxy, sulfo ou carboxypropyloxy et $R_2$ est un atome d'hydrogène, d'halogène, un groupe alkyle en $C_{1-4}$ ou un sel de ce dérivé de benzidine, en présence d'un dérivé de phénylène non chromogène ou peu chromogène de formule générale (II):

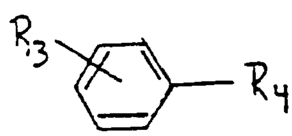

dans laquelle $R_3$ et $R_4$ sont identiques ou différents et représentent un groupe hydroxy, amino, alkyl-($C_{1-4}$)amino ou benzylamino, pour retarder le développement de couleur afin de permettre un point final de dilution net et qu'ensuite l'activité peroxydase est déterminée.

2. Procédure suivant la revendication 1, caractérisée en ce que $R_1$ du dérivé de benzydine (I) est un atome d'hydrogène, un groupe méthyle ou méthoxy et $R_2$ est un atome d'hydrogène ou un groupe méthyle.

3. Procédure suivant la revendication 1, caractérisée en ce que $R_1$ et $R_2$ du dérivé de benzidine (I) sont des groupes méthyles en ortho des groupes amino respectifs.

4. Procédure suivant la revendication 1, caractérisée en ce que le dérivé de phénylène (II) est l'hydroquinone, 1'o-phénylènediamine ou le 4-aminophénol.

5. Procédure suivant la revendication 1, caractérisée en ce que le dérivé de phénylène (II) est l'hydroquinone.

6. Procédure suivant la revendication 1, caractérisée en ce que la couleur est retardée pendant une période de 5 à 60 minutes.

7. Procédure suivant la revendication 6, caractérisée en ce que la couleur est retardée pendant une période de 15 à 45 minutes.

8. Réactif pour la détermination de l'activité peroxydase par titrage à la dilution finale, caractérisé en ce

qu'il comprend un dérivé de benzydine de formule générale (I) :

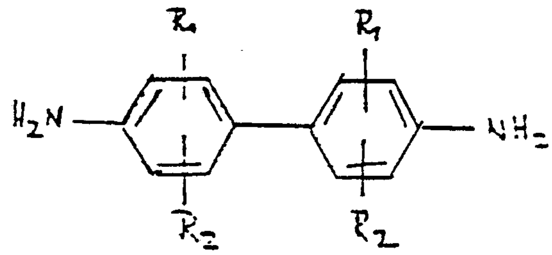

dans laquelle $R_1$ est un atome d'hydrogène, d'halogène, un groupe amino, alkyle en $C_{1-4}$ , alcoxy en $C_{1-4}$ , carboxy, sulfo ou carboxypropyloxy et où $R_2$ est un atome d'hydrogène, d'halogène, un groupe alkyle en $C_{1-4}$ ou un sel de ce dérivé de benzidine, et un dérivé de phénylène de formule généale (II) :

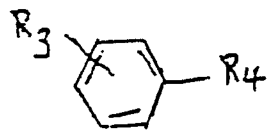

dans laquelle $R_3$ et $R_4$ sont identiques ou différents et représentent un groupe hydroxy, amino, alkyl-($C_{1-4}$)amino ou benzylamino.

**9.** Kit pour déterminer une activité peroxydase par titrage à la dilution finale, caractérisé en ce qu'il comprend :
(a) un dérivé de benzidine de formule générale (I) :

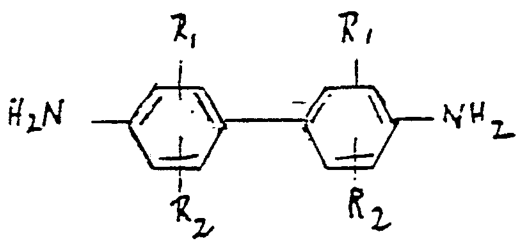

dans laquelle $R_1$ est un atome d'hydrogène, d'halogène, un groupe amino, alkyle en $C_{1-4}$ , alcoxy en $C_{1-4}$ , carboxy, sulfo ou carboxypropyloxy, et $R_2$ est un atome d'hydrogène, d'halogène, un groupe alkyle en $C_{1-4}$ ou un sel de ce dérivé de benzidine,
(b) un dérivé de phénylène de formule générale (II) :

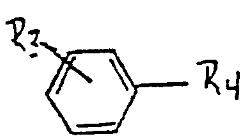

dans laquelle $R_3$ et $R_4$ sont identiques ou différents et représentent un groupe hydroxy, amino, alkyl-($C_{1-4}$)amino ou benzylamino,
(c) du peroxyde d'hydrogène ou un adduct de celui-ci,
(d) un conjugué de peroxydase de raifort et d'un antigène ou d'un anticorps, et
(e) un support solide revêtu d'un antigène ou d'un anticorps qui va réagir avec l'analyte à déterminer.

**Patentansprüche**

1. Verfahren zur Bestimmung der Peroxidase-Aktivität durch Endverdünnungstitration unter für die Peroxidase-Bestimmung durch Endverdünnungstitration üblichen Bedingungen, bei der Peroxidase mit einem Benzidinderivat umgesetzt wird, wobei als Verbesserung zur Durchführung besagten Verfahrens als Benzidinderivat ein Benzidinderivat der allgemeinen Formel (I)

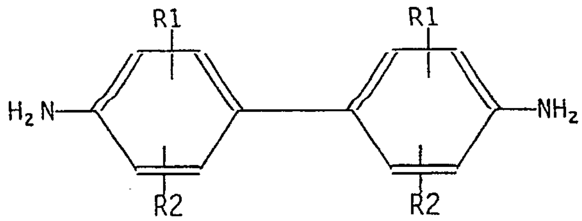

worin
R1 für ein Wasserstoffatom, ein Halogenatom, eine Aminogruppe, eine ($C_1$-$C_4$)-Alkylgruppe, eine ($C_1$-$C_4$)-Alkoxygruppe, eine Carboxygruppe, eine Sulfogruppe oder eine Carboxypropyloxygruppe und
R2 für ein Wasserstoffatom, ein Halogenatom oder eine ($C_1$-$C_4$)-Alkylgruppe stehen, oder ein Salz dieses Benzidinderivats in Gegenwart eines nicht chromogenen oder schwach chromogenen Phenylenderivats der allgemeinen Formel (II)

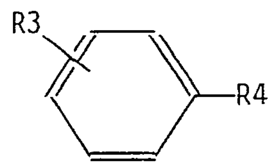

worin
R3 und R4 gleich oder verschieden sind und für eine Hydroxygruppe, eine Aminogruppe, eine ($C_1$-$C_4$)-Alkylaminogruppe oder eine Benzylaminogruppe stehen, benutzt wird, um die Farbentwicklung zum Erhalt eines scharfen Verdünnungsendpunktes zu verzögern und danach die Peroxidase-Aktivität zu bestimmen.

2. Verfahren nach Anspruch 1, wobei der Substituent R1 des Benzidinderivats I für Wasserstoff, Methyl oder Methoxy steht und wobei R2 für Wasserstoff oder Methyl steht.

3. Verfahren nach Anspruch 1, wobei die Substituenten R1 und R2 des Benzidinderivats I für Methyl in ortho-Stellung zu den entsprechenden Aminogruppen stehen.

4. Verfahren nach Anspruch 1, wobei das Phenylenderivat II Hydrochinon, o-Phenylendiamin oder 4-Aminophenol ist.

5. Verfahren nach Anspruch 1, wobei das Phenylenderivat II Hydrochinon ist.

6. Verfahren nach Anspruch 1, wobei die Farbentwicklung für eine Zeitspanne von 5 bis 60 Minuten verzögert wird.

7. Verfahren nach Anspruch 6, wobei die Farbentwicklung für eine Zeitspanne von 15 bis 45 Minuten verzögert wird.

8. Reagenz für die Bestimmung von Peroxidase durch Endverdünnungstitration beinhaltend ein Benzidinderivat der allgemeinen Formel (I)

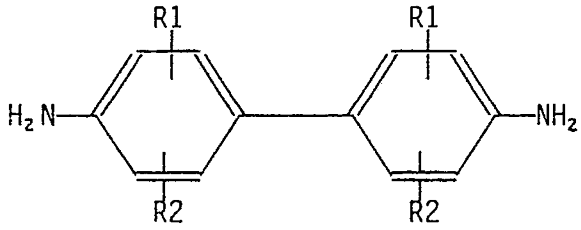

worin
R1 für ein Wasserstoffatom, ein Halogenatom, eine Aminogruppe, eine ($C_1$-$C_4$)-Alkylgruppe, eine ($C_1$-$C_4$)-Alkoxygruppe, eine Carboxygruppe, eine Sulfogruppe oder eine Carboxylpropyloxygruppe und
R2 für ein Wasserstoffatom, ein Halogenatom oder eine ($C_1$-$C_4$)-Alkylgruppe stehen, oder ein Salz dieses Benzidinderivats und ein Phenylenderivat der allgemeinen Formel (II)

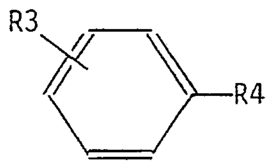

worin
R3 und R4 gleich oder verschieden sind und für eine Hydroxygruppe, eine Aminogruppe, eine ($C_1$-$C_4$)-Alkylaminogruppe oder eine Benzylaminogruppe stehen.

**9.** Kit zur Bestimmung der Peroxidase-Aktivität durch Endverdünnungstitration beinhaltend
(a) ein Benzidinderivat der allgemeinen Formel (I)

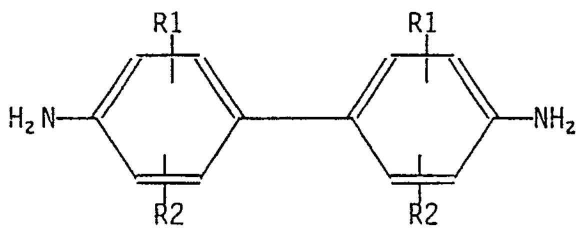

wobei
R1 für ein Wasserstoffatom, ein Halogenatom, eine Aminogruppe, eine ($C_1$-$C_4$)-Alkylgruppe, eine ($C_1$-$C_4$)-Alkoxygruppe, eine Carboxygruppe, eine Sulfogruppe oder eine Carboxypropyloxygruppe und
R2 für ein Wasserstoffatom, ein Halogenatom oder eine ($C_1$-$C_4$)-Alkylgruppe stehen, oder ein Salz dieses Benzidinderivats,
(b) ein Phenylenderivat der allgemeinen Formel (II)

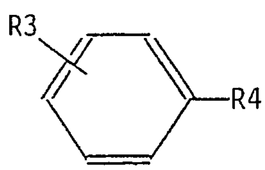

wobei
R3 und R4 gleich oder verschieden sind und für eine Hydroxygruppe, eine Aminogruppe, eine ($C_1$-$C_4$)-Alkylaminogruppe oder eine Benzylaminogruppe stehen,
(c) Wasserstoffperoxid oder eine Anlagerungsverbindung davon,
(d) ein Konjugat von Meerrettichperoxidase mit einem Antigen oder Antikörper und

(e) einen mit einem mit dem zu bestimmenden Analyten reagierenden Antigen oder Antikörper beschichteten festen Träger.

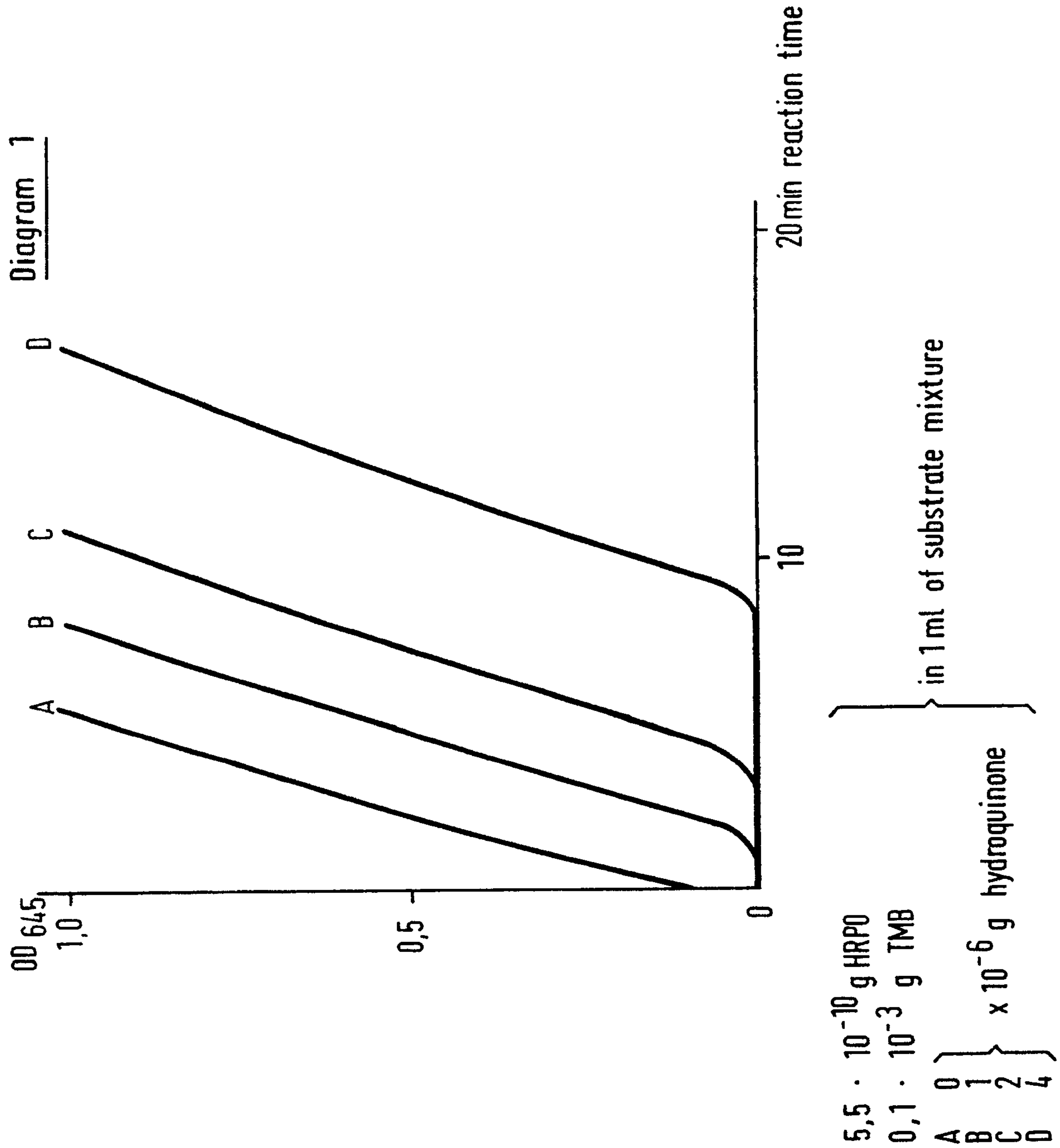
Diagram 1
OD 645
1,0
0,5
0
10
20 min reaction time
A
B
C
D
5,5 · $10^{-10}$ g HRPO
0,1 · $10^{-3}$ g TMB
A 0
B 1
C 2
D 4
x $10^{-6}$ g hydroquinone
in 1 ml of substrate mixture

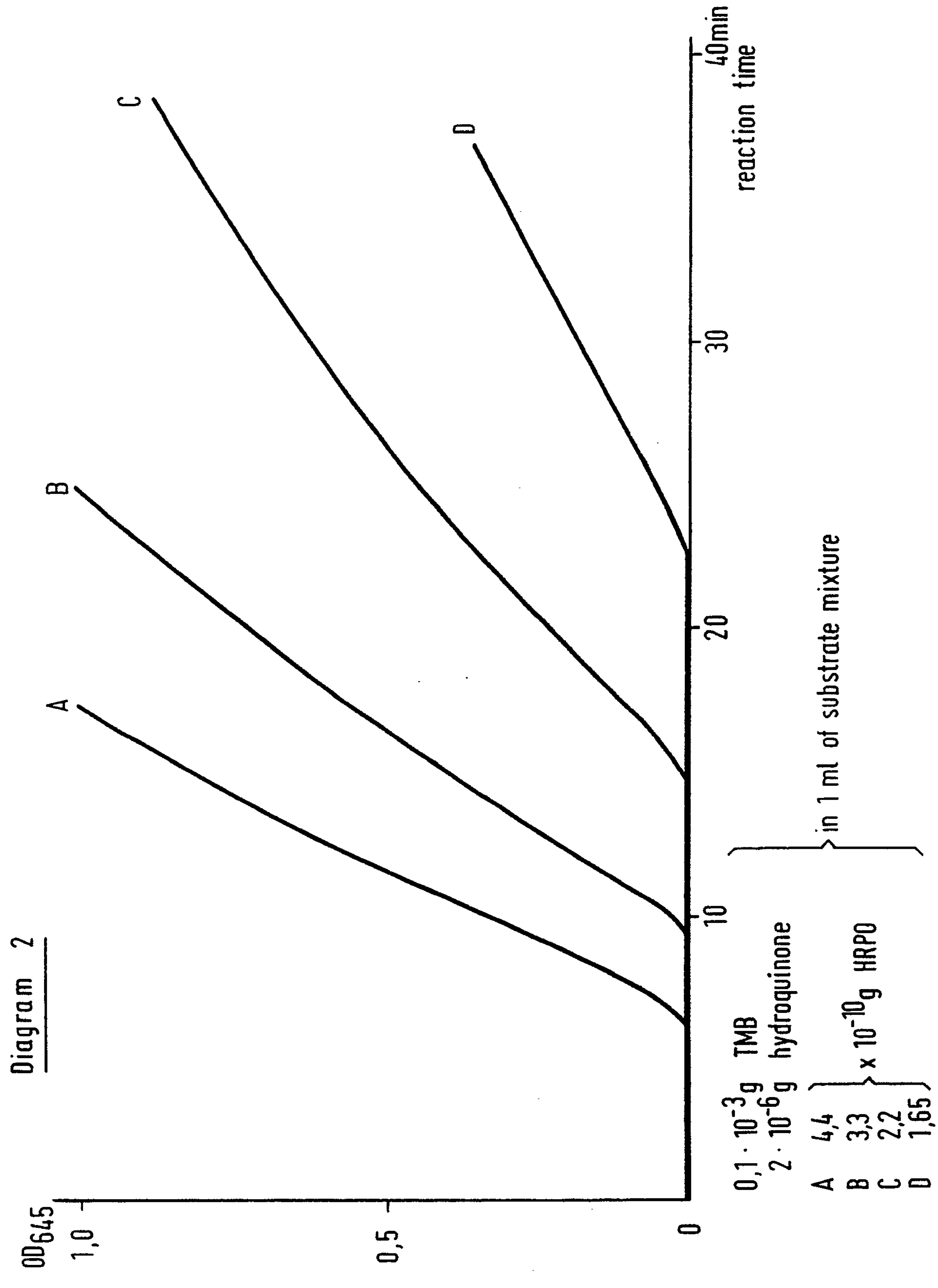
Diagram 2
OD645
1,0
0,5
0
A
B
C
D
10
20
30
40min
reaction time
0,1 · 10^-3 g TMB
2 · 10^-6 g hydroquinone
A 4,4
B 3,3
C 2,2
D 1,65
x 10^-10 g HRPO
in 1 ml of substrate mixture